# EUROPEAN PATENT SPECIFICATION

(11) **EP 1 074 624 B1**
(45) Date of publication and mention of the grant of the patent: **13.04.2005**
(21) Application number: 99973716.6
(22) Date of filing: 05.10.1999
(51) Int. Cl.: C12N 15/38, C12N 5/06, C12N 7/08, C12P 21/08, C12Q 1/68, C12Q 1/70, C07K 14/04, C07K 16/08, A61K 39/25, A61K 39/12

(54) **ATTENUATED CHICKEN-POX VIRUS OKA STRAIN GENE 62 AND METHOD FOR IDENTIFYING VIRUS STRAIN FOR ATTENUATED LIVE CHICKEN-POX VACCINE BY USING THE GENE**
GEN 62 DES ATTENUIERTEN WINDPOCKEN-VIRUSSTAMMES OKA UND VERFAHREN ZUR IDENTIFIKATION EINES ATTENUIERTEN, LEBENDEN WINDPOCKENVIRUSSTAMMES UNTER VERWENDUNG DIESES GENES.
GENE 62 DE LA SOUCHE OKA DU VIRUS DE LA VARICELLE ATTENUE ET PROCEDE D'IDENTIFICATION DE LA SOUCHE DU VIRUS POUR LE VACCIN DE LA VARICELLE VIVANT ATTENUE A L'AIDE DE CE GENE

(30) Priority: 25.02.1999 JP 4896499
(43) Date of publication of application: 07.02.2001
(73) Proprietor: The Research Foundation for Microbial Diseases of Osaka University, Suita-shi, Osaka 565-0871 (JP)
(72) Inventor: GOMI, Yasuyuki, Kanonji Institute, Kanonji-shi, Kagawa 768-0061 (JP); IMAGAWA, Tadashi, Kanonji Institute, Kanonji-shi, Kagawa 768-0061 (JP); OSAME, Juichiro, Kanonji Institute, Kanonji-shi, Kagawa 768-0061 (JP); TAKAHASHI, Toshiaki, Research Foundation, Suita-shi, Osaka 565-0871 (JP); YAMANISHI, Koichi, Osaka 563-0104 (JP)
(74) Representative: Webber, Philip Michael
(86) International application number: PCT/JP1999/005476
(87) International publication number: WO 2000/050603

(56) References cited:
- EP-A1- 0 510 996
- WO-A1-97/43420
- US-A- 3 985 615
- MORI CHISATO ET AL: "Identification of the Oka strain of the live attenuated varicella vaccine from other clinical isolates by molecular epidemiologic analysis." JOURNAL OF INFECTIOUS DISEASES, vol. 178, no. 1, July 1998 (1998-07), pages 35-38, XP002193508 ISSN: 0022-1899
- LARUSSA PHILIP ET AL: "Polymerase chain reaction and restriction fragment length polymorphism analysis of varicella-zoster virus isolates from the United States and other parts of the world." JOURNAL OF INFECTIOUS DISEASES, vol. 178, no. SUPPL. 1, November 1998 (1998-11), pages S64-S66, XP002193509 ISSN: 0022-1899
- SCHOONBROODT SONIA ET AL: "Enhancement of Varicella-Zoster virus infection in cell lines expressing ORF4- or ORF62-encoded proteins." JOURNAL OF MEDICAL VIROLOGY, vol. 49, no. 4, 1996, pages 264-273, XP002193510 ISSN: 0146-6615
- GOMI YASUYUKI ET AL: "Oka varicella vaccine is distinguishable from its parental virus in DNA sequence of open reading frame 62 and its transactivation activity." JOURNAL OF MEDICAL VIROLOGY, vol. 61, no. 4, August 2000 (2000-08), pages 497-503, XP001056668 ISSN: 0146-6615
- ARGAW TAKELE ET AL: "Nucleotide sequences that distinguish Oka vaccine from parental Oka and other varicella-zoster virus isolates." JOURNAL OF INFECTIOUS DISEASES, vol. 181, no. 3, March 2000 (2000-03), pages 1153-1157, XP002193511 ISSN: 0022-1899
- FORGHANI B. ET AL.: 'Monoclonal antibody to immediate early protein encoded by varicella-zoster virus gene 62' VIRUS RES., vol. 16, no. 2, 1990, pages 195 - 210, XP002925240
- DAVISON A.J. ET AL.: 'The complete DNA sequence of varicella-zoster virus' J. GEN. VIROL., vol. 67, no. 9, 1986, pages 1759 - 1816, XP002925241

### Sequence Listing

<110> The Research Foundation for Microbial Diseases of Osaka University
   <120> Gene 62 of Oka vaccine virus and method for identifying virus strain for live attenuated vaccine virus using the gene 62
   <140> PCT/JP99/05476
   <141> 1999-10-05
   <150> JP11-48964
   <151> 1999-02-25
   <160> 12
<210> 1
   <211> 4226
   <212> DNA
   <213> Varicella-Zoster virus
   <220>
   <221> CDS
   <222> 229..4158
<400> 1
<210> 2
   <211> 18
   <212> DNA
   <213> Artificial Sequence
   <220>
   <223> Description of the artificial sequence: PCR primer
<400> 2
   gatcaaagct tagcgcag    18
<210> 3
   <211> 18
   <212> DNA
   <213> Artificial Sequence
   <220>
   <223> Description of the artificial sequence: PCR primer
<400> 3
   cctatagcat ggctccag 18
<210> 4
   <211> 1310
   <212> PRT
   <213> Varicella-Zoster virus
<400> 4
   Met Asp Thr Pro Pro Met Gln Arg Ser Thr Pro Gln Arg Ala Gly Ser
   1 5 10 15
   Pro Asp Thr Leu Glu Leu Met Asp Leu Leu Asp Ala Ala Ala Ala Ala
   20 25 30
   Ala Glu His Arg Ala Arg Val Val Thr Ser Ser Gln Pro Asp Asp Leu
   35 40 45
   Leu Phe Gly Glu Asn Gly Val Met Val Gly Arg Glu His Glu Ile Val
   50 55 60
   Ser Ile Pro Ser Val Ser Gly Leu Gln Pro Glu Pro Arg Thr Glu Asp
   65 70 75 80
   Val Gly Glu Glu Leu Thr Gln Asp Asp Tyr Val Cys Glu Asp Gly Gln
   85 90 95
   Asp Leu Met Gly Ser Pro Val Ile Pro Leu Ala Glu Val Phe His Thr
   100 105 110
   Arg Phe Ser Glu Ala Gly Ala Arg Glu Pro Thr Gly Ala Asp Arg Ser
   115 120 125
   Leu Glu Thr Val Ser Leu Gly Thr Lys Leu Ala Arg Ser Pro Lys Pro
   130 135 140
   Pro Met Asn Asp Gly Glu Thr Gly Arg Gly Thr Thr Pro Pro Phe Pro
   145 150 155 160
   Gln Ala Phe Ser Pro Val Ser Pro Ala Ser Pro Val Gly Asp Ala Ala
   165 170 175
   Gly Asn Asp Gln Arg Glu Asp Gln Arg Ser Ile Pro Arg Gln Thr Thr
   180 185 190
   Arg Gly Asn Ser Pro Gly Leu Pro Ser Val Val His Arg Asp Arg Gln
   195 200 205
   Thr Gln Ser Ile Ser Gly Lys Lys Pro Gly Asp Glu Gln Ala Gly His
   210 215 220
   Ala His Ala Ser Gly Asp Gly Val Val Leu Gln Lys Thr Gln Arg Pro
   225 230 235 240
   Ala Gln Gly Lys Ser Pro Lys Lys Lys Thr Leu Lys Val Lys Val Pro
   245 250 255
   Leu Pro Ala Arg Lys Pro Gly Gly Pro Val Pro Gly Pro Val Glu Gln
   260 265 270
   Leu Tyr His Val Leu Ser Asp Ser Val Pro Ala Lys Gly Ala Lys Ala
   275 280 285
   Asp Leu Pro Phe Glu Thr Asp Asp Thr Arg Pro Arg Lys His Asp Ala
   290 295 300
   Arg Gly Ile Thr Pro Arg Val Pro Gly Arg Ser Ser Gly Gly Lys Pro
   305 310 315 320
   Arg Ala Phe Leu Ala Leu Pro Gly Arg Ser His Ala Pro Asp Pro Ile
   325 330 335
   Glu Asp Asp Ser Pro Val Glu Lys Lys Pro Lys Ser Arg Glu Phe Val
   340 345 350
   Ser Ser Ser Ser Ser Ser Ser Ser Trp Gly Ser Ser Ser Glu Asp Glu
   355 360 365
   Asp Asp Glu Pro Arg Arg Val Ser Val Gly Ser Glu Thr Thr Gly Ser
   370 375 380
   Arg Ser Gly Arg Glu His Ala Pro Ser Pro Ser Asn Ser Asp Asp Ser
   385 390 395 400
   Asp Ser Asn Asp Gly Gly Ser Thr Lys Gln Asn Ile Gln Pro Gly Tyr
   405 410 415
   Arg Ser Ile Ser Gly Pro Asp Pro Arg Ile Arg Lys Thr Lys Arg Leu
   420 425 430
   Ala Gly Glu Pro Gly Arg Gln Arg Gln Lys Ser Phe Ser Leu Pro Arg
   435 440 445
   Ser Arg Thr Pro Ile Ile Pro Pro Val Ser Gly Pro Leu Met Met Pro
   450 455 460
   Asp Gly Ser Pro Trp Pro Gly Ser Ala Pro Leu Pro Ser Asn Arg Val
   465 470 475 480
   Arg Phe Gly Pro Ser Gly Glu Thr Arg Glu Gly His Trp Glu Asp Glu
   485 490 495
   Ala Val Arg Ala Ala Arg Ala Arg Tyr Glu Ala Ser Thr Glu Pro Val
   500 505 510
   Pro Leu Tyr Val Pro Glu Leu Gly Asp Pro Ala Arg Gln Tyr Arg Ala
   515 520 525
   Leu Ile Asn Leu Ile Tyr Cys Pro Asp Arg Asp Pro Ile Ala Trp Leu
   530 535 540
   Gln Asn Pro Lys Leu Thr Gly Val Asn Ser Ala Leu Asn Gln Phe Tyr
   545 550 555 560
   Gln Lys Leu Leu Pro Pro Gly Arg Ala Gly Thr Ala Val Thr Gly Ser
   565 570 575
   Val Ala Ser Pro Val Pro His Val Gly Glu Ala Met Ala Thr Gly Glu
   580 585 590
   Ala Leu Trp Ala Leu Pro His Ala Ala Ala Ala Val Ala Met Ser Arg
   595 600 605
   Arg Tyr Asp Arg Ala Gln Lys His Phe Ile Leu Gln Ser Leu Arg Arg
   610 615 620
   Ala Phe Ala Ser Met Ala Tyr Pro Glu Ala Thr Gly Ser Ser Pro Ala
   625 630 635 640
   Ala Arg Ile Ser Arg Gly His Pro Ser Pro Thr Thr Pro Ala Thr Gln
   645 650 655
   Thr Pro Asp Pro Gln Pro Ser Ala Ala Ala Arg Ser Leu Ser Val Cys
   660 665 670
   Pro Pro Asp Asp Arg Leu Arg Thr Pro Arg Lys Arg Lys Ser Gln Pro
   675 680 685
   Val Glu Ser Arg Ser Leu Leu Asp Lys Ile Arg Glu Thr Pro Val Ala
   690 695 700
   Asp Ala Arg Val Ala Asp Asp His Val Val Ser Lys Ala Lys Arg Arg
   705 710 715 720
   Val Ser Glu Pro Val Thr Ile Thr Ser Gly Pro Val Val Asp Pro Pro
   725 730 735
   Ala Val Ile Thr Met Pro Leu Asp Gly Pro Ala Pro Asn Gly Gly Phe
   740 745 750
   Arg Arg Ile Pro Arg Gly Ala Leu His Thr Pro Val Pro Ser Asp Gln
   755 760 765
   Ala Arg Lys Ala Tyr Cys Thr Pro Glu Thr Ile Ala Arg Leu Val Asp
   770 775 780
   Asp Pro Leu Phe Pro Thr Ala Trp Arg Pro Ala Leu Ser Phe Asp Pro
   785 790 795 800
   Gly Ala Leu Ala Glu Ile Ala Ala Arg Arg Pro Gly Gly Gly Asp Arg
   805 810 815
   Arg Phe Gly Pro Pro Ser Gly Val Glu Ala Leu Arg Arg Arg Cys Ala
   820 825 830
   Trp Met Arg Gln Ile Pro Asp Pro Glu Asp Val Arg Leu Leu Ile Ile
   835 840 845
   Tyr Asp Pro Leu Pro Gly Glu Asp Ile Asn Gly Pro Leu Glu Ser Thr
   850 855 860
   Leu Ala Thr Asp Pro Gly Pro Ser Trp Ser Pro Ser Arg Gly Gly Leu
   865 870 875 880
   Ser Val Val Leu Ala Ala Leu Ser Asn Arg Leu Cys Leu Pro Ser Thr
   885 890 895
   His Ala Trp Ala Gly Asn Trp Thr Gly Pro Pro Asp Val Ser Ala Leu
   900 905 910
   Asn Ala Arg Gly Val Leu Leu Leu Ser Thr Arg Asp Leu Ala Phe Ala
   915 920 925
   Gly Ala Val Glu Tyr Leu Gly Ser Arg Leu Ala Ser Ala Arg Arg Arg
   930 935 940
   Leu Leu Val Leu Asp Ala Val Ala Leu Glu Arg Trp Pro Arg Asp Gly
   945 950 955 960
   Pro Ala Leu Ser Gln Tyr His Val Tyr Val Arg Ala Pro Ala Arg Pro
   965 970 975
   Asp Ala Gln Ala Val Val Arg Trp Pro Asp Ser Ala Val Thr Glu Gly
   980 985 990
   Leu Ala Arg Ala Val Phe Ala Ser Ser Arg Thr Phe Gly Pro Ala Ser
   995 1000 1005
   Phe Ala Arg Ile Glu Thr Ala Phe Ala Asn Leu Tyr Pro Gly Glu
   1010 1015 1020
   Gln Pro Leu Cys Leu Cys Arg Gly Gly Asn Val Ala Tyr Thr Val
   1025 1030 1035
   Cys Thr Arg Ala Gly Pro Lys Thr Arg Val Pro Leu Ser Pro Arg
   1040 1045 1050
   Glu Tyr Arg Gln Tyr Val Leu Pro Gly Phe Asp Gly Cys Lys Asp
   1055 1060 1065
   Leu Ala Arg Gln Ser Arg Gly Leu Gly Leu Gly Ala Ala Asp Phe
   1070 1075 1080
   Val Asp Glu Ala Ala His Ser His Arg Ala Ala Asn Arg Trp Gly
   1085 1090 1095
   Leu Gly Ala Ala Leu Arg Pro Val Phe Leu Pro Glu Gly Arg Arg
   1100 1105 1110
   Pro Gly Ala Ala Gly Pro Glu Ala Gly Asp Val Pro Thr Trp Ala
   1115 1120 1125
   Arg Val Phe Cys Arg His Ala Leu Leu Glu Pro Asp Pro Ala Ala
   1130 1135 1140
   Glu Pro Leu Val Leu Pro Pro Val Ala Gly Arg Ser Val Ala Leu
   1145 1150 1155
   Tyr Ala Ser Ala Asp Glu Ala Arg Asn Ala Leu Pro Pro Ile Pro
   1160 1165 1170
   Arg Val Met Trp Pro Pro Gly Phe Gly Ala Ala Glu Thr Val Leu
   1175 1180 1185
   Glu Gly Ser Asp Gly Thr Arg Phe Val Phe Gly His His Gly Gly
   1190 1195 1200
   Ser Glu Arg Pro Ala Glu Thr Gln Ala Gly Arg Gln Arg Arg Thr
   1205 1210 1215
   Ala Asp Asp Arg Glu His Ala Leu Glu Pro Asp Asp Trp Glu Val
   1220 1225 1230
   Gly Cys Glu Asp Ala Trp Asp Ser Glu Glu Gly Gly Gly Asp Asp
   1235 1240 1245
   Gly Asp Ala Pro Gly Ser Ser Phe Gly Val Ser Ile Val Ser Val
   1250 1255 1260
   Ala Pro Gly Val Leu Arg Asp Arg Arg Val Gly Leu Arg Pro Ala
   1265 1270 1275
   Val Lys Val Glu Leu Leu Ser Ser Ser Ser Ser Ser Glu Asp Glu
   1280 1285 1290
   Asp Asp Val Trp Gly Gly Arg Gly Gly Arg Ser Pro Pro Gln Ser
   1295 1300 1305
   Arg Gly
   1310
<210> 5
   <211> 37
   <212> DNA
   <213> Artificial/Unknown
<220>
   <221> misc_feature
   <222> (1)..(37)
   <223> Primer derived from Dumas strain (J.Gen.Virol.,67:1759-1816,1986)
<400> 5
   ccgagctcga attctagatt cataaaaacc gttccgc 37
<210> 6
   <211> 38
   <212> DNA
   <213> Artificial/Unknown
<220>
   <221> misc_feature
   <222> (1)..(19)
   <223> M13 forward primer sequence
<220>
   <221> misc_feature
   <222> (20)..(38)
   <223> Primer derived from Dumas strain (J.Gen.Virol.,67:1759-1816,1986)
<400> 6
   tttcccagtc acgacgttgt tcataaaaac cgttccgc 38
<210> 7
   <211> 40
   <212> DNA
   <213> Artificial/Unknown
<220>
   <221> misc_feature
   <222> (1)..(19)
   <223> M13 forward primer sequence
<220>
   <221> misc_feature
   <222> (20) .. (40)
   <223> Primer derived from Dumas strain (J.Gen.Virol.,67:1759-1816,1986)
<400> 7
   tttcccagtc acgacgttgc aggcacaacc ggttactcag 40
<210> 8
   <211> 40
   <212> DNA
   <213> Artificial/Unknown
<220>
   <221> misc_feature
   <222> (1)..(19)
   <223> M13 forward primer sequence
<220>
   <221> misc_feature
   <222> (20)..(40)
   <223> Primer derived from Dumas strain (J.Gen.Virol.,67:1759-1816,1986)
<400> 8
   tttcccagtc acgacgttgt ttggtcttac gaatcctcgg 40
<210> 9
   <211> 40
   <212> DNA
   <213> Artificial/Unknown
<220>
   <221> misc_feature
   <222> (1)..(40)
   <223> Primer derived from Dumas strain (J.Gen.Virol.,67:1759-1816,1986)
<400> 9
   acctgatcag aattctgcag agcggtctct ccttaaacgc 40
<210> 10
   <211> 40
   <212> DNA
   <213> Artificial/Unknown
<220>
   <221> misc_feature
   <222> (1)..(20)
   <223 > M13 reverse primer sequence
<220>
   <221> misc_feature
   <222> (21) .. (40)
   <223> Primer derived from Dumas strain (J.Gen.Virol.,67:1759-1816,1986)
<400> 10
   ggataacaat ttcacacagg ttctgatcat ctacgatccg 40
<210> 11
   <211> 40
   <212> DNA
   <213> Artificial/Unknown
<220>
   <221> misc_feature
   <222> (1)..(20)
   <223> M13 reverse primer sequence
<220>
   <221> misc_feature
   <222> (21)..(40)
   <223> Primer derived from Dumas strain (J.Gen.Virol.,67:1759-1816,1986)
<400> 11
   ggataacaat ttcacacagg caaattcgga tgattcggac 40
<210> 12
   <211> 40
   <212> DNA
   <213> Artificial/Unknown
<220>
   <221> misc_feature
   <222> (1)..(20)
   <223> M13 reverse primer sequence
<220>
   <221> misc_feature
   <222> (21)..(40)
   <223> Primer derived from Dumas strain (J.Gen.Virol.,67:1759-1816,1986)
<400> 12
   ggataacaat ttcacacagg agcggtctct ccttaaacgc 40

## Description

### Technical Field

The present application relates to gene 62 of the Oka vaccine virus and a method for identifying a virus strain for live attenuated varicella-zoster vaccine (referred to as "attenuated varicella-zoster virus strain" hereinafter) using the DNA sequence of gene 62. More specifically, the application relates to the gene 62 for use in discriminating the Oka vaccine virus and an attenuated varicella-zoster virus strain acceptable as the effective component of live attenuated vaccine for varicella-zoster, from the wild-type Oka vaccine virus (referred to as "virulent parental strain" or "parental strain" hereinafter) and other wild-type varicella-zoster virus strains; and a method for identifying an attenuated varicella-zoster virus, based on the nucleotide sequence of the gene 62.

### Background Art

Varicella-zoster virus (abbreviated as VZV; referred to as "varicella virus" or "VZV" hereinafter) is the pathogenic virus of varicella and zoster in humans. Virus derived from "Oka vaccine virus" (Japanese Patent Publication No. 41202/1978; Lancet 2: 1288-1290, 1974) as one attenuated varicella-zoster virus strain has been used as the single unique seed for preparing live attenuated varicella vaccines, which are widely used worldwide [Requirements for Varicella Vaccine (Live) adopted 1984: WHO Technical Report Series, No. 725, pp. 102-124, 1985].

Currently, the safety and effectiveness of the live attenuated varicella vaccines is certificated by the regulation of the passage number of the varicella seed virus with approval of the preparation (seed lot system). More specifically, so as to avoid the risk of genetic modification of the attenuated virus over continuous passages of the seed virus so that the resulting virus resumes the pathogenicity, it is defined under regulation that under provision that the passage number of the seed when approved is designated 0, virus within the total passage number of 10 from the passage number 0 is essentially used for such vaccines. In other words, the quality control and quality certificate of the live attenuated varicella vaccines depend on the observance of the seed lot system by each vaccine manufacture.

Meanwhile, no clinical reaction emerges in healthy children after the inoculation of the live attenuated varicella vaccines. The onset of varicella or zoster only rarely occurs in inoculated subjects with disorders of the immune function. However, whether or not such clinical reaction is due to the vaccination or spontaneous infection with wild-type varicella virus has never been identified. Thus, it is important epidemiologically to analyze the differentiation of the Oka vaccine virus as the seed virus of the live attenuated varicella vaccine from other wild-type VZV strains. Hence, a number of methods for the analysis have been proposed. Since the structure of the VZV genomic genes was reported (Journal of General Virology, 67: 1759-1816, 1986), methods have been proposed, for example, on the basis of the difference in DNA nucleotide sequence among VZV strains (Journal of Virology, 59: 660-668, 1986), the presence or absence of restriction enzyme PstI site (Japanese Journal of Experimental Medicine, 59: 233-237, 1989), determination based on RFLP (restriction fragment length polymorphism) by PCR (polymerase chain reaction) (Journal of Virology, 66: 1016-1020, 1992), and a combination of the presence or absence of the PstI site and the RFLP analysis of PCR products (Journal of Clinical Microbiology, 33:658-660, 1995). However, these methods are at low reliability when used singly. Thus, it has been difficult to definitely differentiate the Oka vaccine virus from other wild-type VZV strains.

Alternatively, the applicants of the present application have made diverse gene analysis of the Oka vaccine virus and other varicella virus strains (for example, known wild-type strains and wild-type strains newly isolated from patients with spontaneously infected varicella, and the like), and have identified eight requirements to differentiate the two kinds of strain types. Further the applicants have found that an unknown virus is acceptable as live varicella vaccine only when the virus satisfies all the eight requirements, and they have made a patent application based on the finding (WO 97/43420; referred to as "previous invention" hereinafter).

The method of the previous invention can discriminate the Oka vaccine virus from other varicella-zoster virus strains, but can never discriminate the Oka vaccine virus from the parental strain. Because all the eight required indicators are essentially examined, disadvantageously, the method needs a lot of time and labors until the discrimination is established.

The inventors of the present application have made investigations about a method for possibly differentiating the Oka vaccine virus from the parental strain, and they have found that the Oka vaccine virus, the parental strain and other wild-type varicella virus strains can be differentiated from each other in a simple and reliable manner, by detecting the mutation of varicella virus gene 62.

The invention of the present application has been attained on the basis of such novel finding, and a purpose of the invention is to provide the Oka vaccine virus-specific gene 62.

It is an additional purpose of the invention to provide the protein encoded by the gene 62 and a peptide thereof, an antibody and cytotoxic T lymphocyte (abbreviated as CTL) preparable by using them as antigen.

It is a still additional purpose of the invention to provide a method for identifying an attenuated varicella-zoster virus strain using the presence or absence of the mutation of the gene 62 as an indicator.

### Disclosure of Invention

The present application provides the following individual inventions to satisfy the aforementioned purposes.
(1) Gene 62 of Oka vaccine virus, which comprises the following base substitutions (a) to (d) in the nucleotide sequence of SEQ ID No. 1:
   (a) the base A at position 2110 is G;
   (b) the base A at position 3100 is G;
   (c) the base T at position 3818 is C; and
   (d) the base A at position 4006 is G.
(2) The gene 62 of the Oka vaccine virus of the above (1), which comprises one or more of the following base substitutions (e) to (g) in addition to the base substitutions (a) to (d):
   (e) the base A at position 1251 is G;
   (f) the base A at position 2226 is G; and
   (g) the base A at position 3657 is G.
(3) The gene 62 of the Oka vaccine virus of the above (2), which comprises one or more of the following base substitutions (h) to (o), in addition to the base substitutions (a) to (d) and one or more of the base substitutions (e) to (g):
   (h) the base T at position 162 is C;
   (i) the base T at position 225 is C;
   (j) the base T at position 523 is C;
   (k) the base T at position 1565 is C;
   (l) the base T at position 1763 is C;
   (m) the base T at position 2652 is C;
   (n) the base T at position 4052 is C; and
   (o) the base T at position 4193 is C.
(4) A fragment of DNA encoding the gene 62 of any one of above (1) to (3), which includes the base-substituted portions, and which can be used as a probe for differentiating the attenuated varicella-zoster virus strains from wild-type varicella-zoster virus strains.
(5) A protein encoded by the gene 62 of the Oka vaccine virus of above (1), which comprises the following amino acid substitutions (A) to (D) in the amino acid sequence of SEQ ID No. 1:
   (A) the amino acid residue Ser at position 628 is Gly;
   (B) the amino acid residue Arg at position 958 is Gly;
   (C) the amino acid residue Val at position 1197 is Ala; and
   (D) the amino acid residue Ile at position 1260 is Val.
(6) The protein of the above (5), which comprises one or more of the following amino acid substitutions (E) to (H) in addition to the amino acid substitutions (A) to (D):
   (E) the amino acid residue Met at position 99 is substituted with Thr;
   (F) the amino acid residue Leu at position 446 is Pro;
   (G) the amino acid residue Val at position 512 is Ala; and
   (H) the amino acid residue Leu at position 1275 is Ser.
(7) A peptide as a part of the protein of the above (5) or (6), which includes the amino acid-substituted portions, and which can be used as an antigen for preparing an antibody or a cytotoxic T lymphocyte which recognises the protein of (5) or (6) but not the protein encoded by gene 62 of wild-type varicella-zoster virus strains.
(8) An antibody or a cytotoxic T lymphocyte which recognises the protein of the above (5) or (6) or the peptide of the above (7) as an antigen, but does not recognise the protein of gene 62 of wild-type varicella-zoster virus strains.
(10) A method for identifying the Oka vaccine virus or an attenuated varicella-zoster virus strain, comprising sequencing the gene 62 of varicella-zoster viruses, and selecting a varicella-zoster virus with the same base substitutions in the gene 62 as the gene 62 of any one of the above (1) to (3).
(11) A method for identifying the Oka vaccine virus or an attenuated varicella-zoster virus strain, comprising selecting a varicella-zoster virus carrying genomic DNA to which the DNA fragment of the above (4) hybridizes.
(12) A method for identifying the Oka vaccine virus or an attenuated varicella-zoster virus strain, comprising selecting a varicella-zoster virus producing an antigen recognized by the antibody or the cytotoxic T lymphocyte of the above (8).
(13) A method for identifying the Oka vaccine virus or an attenuated varicella-zoster virus strain, comprising digesting a DNA fragment comprising at least from 2107th to 2229th in the nucleotide sequence of SEQ ID No. 1 by using restriction enzymes *Nae*I and *Bss*HII, and selecting a varicella-zoster virus from which the DNA fragment is digested into 2 or 3 fragments.
(14) A method according to the above (13), wherein the DNA sequence is prepared by PCR using the oligonucleotides of the nucleotide sequence of SEQ ID No. 2 and 3 as primers and varicella-zoster virus gene 62 as a template.

### Brief Description of Drawings

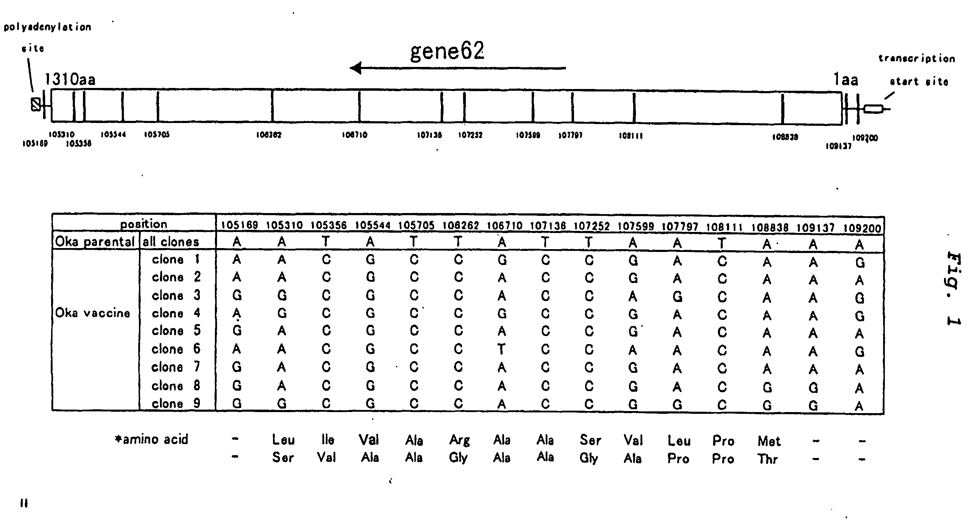

Fig. 1, upper column depicts the schematic view of the structure of varicella virus gene 62; Fig. 1, lower column collectively depicts the sequencing results of the 9 clones derived from the gene 62 of Oka vaccine virus and the sequencing results of the gene 62 of the virulent parental strain.

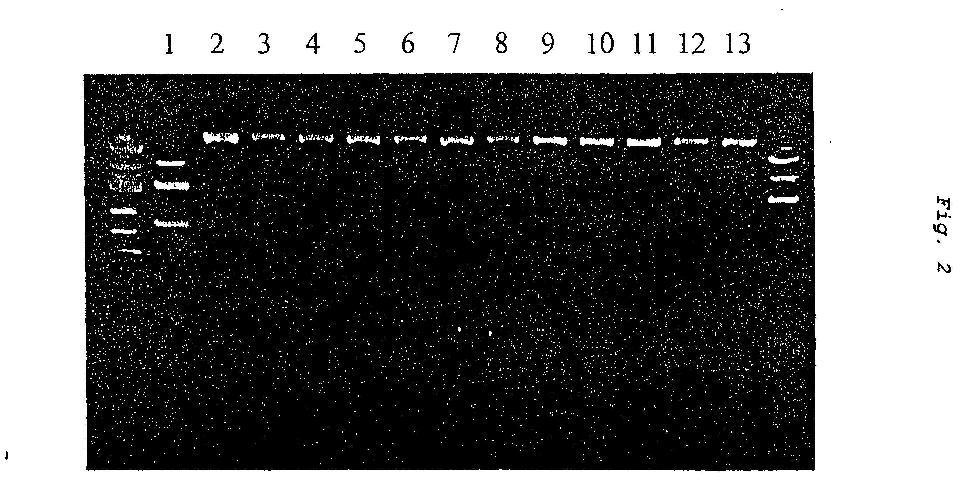

Fig. 2 is the agarose gel electrophoresis charts of the RFLP results of the PCR products of the gene 62 of each of the following varicella-zoster virus strains: Lane 1 for the Oka vaccine virus; Lane 2 for the highly toxic parental strain; Lane 3 for Kawaguchi strain; Lanes 4 to 8 for varicella-derived strain; Lanes 9 to 13 for zoster-derived strain; the lanes on both the sides are for size markers.

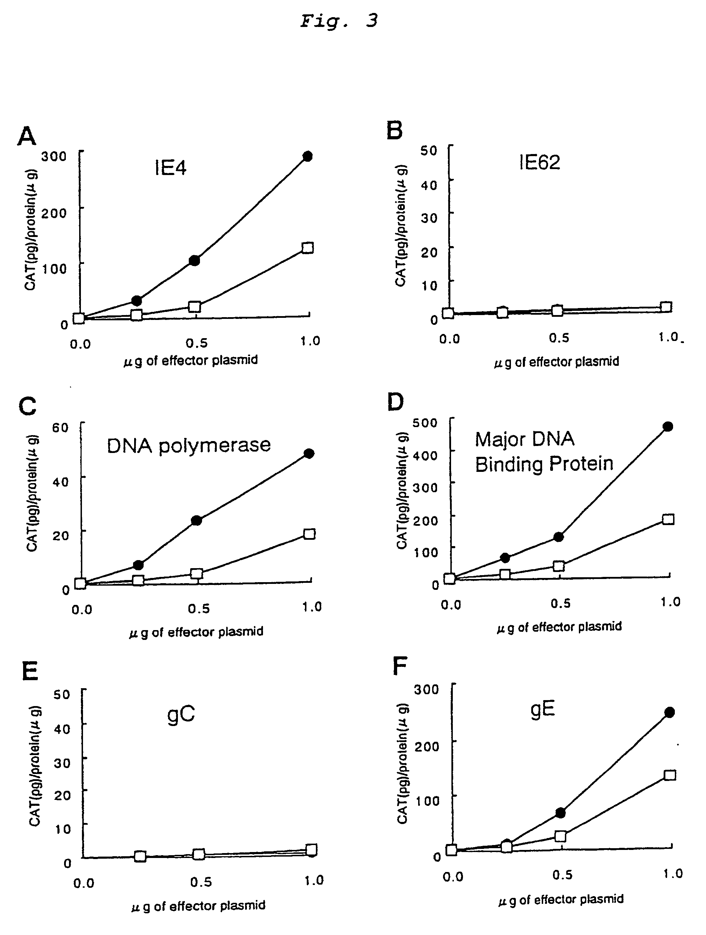

Fig. 3 depicts the results of CAT assay to test transactivation activity of gene 62 products (IE62) of the Oka vaccine virus and virulent Oka parental strain on individual promoters; Fig. 3A shows that IE62s expressed from the effector plasmids transactivate the gene 4 (IE4) promoter in a dose-dependent fashion, wherein the CV1 cell is cotransfected with pCAT-IE4 at a constant amount (0.25µg) and pVac-G62 (open square) or pPar-G62 (closed circle) at various amounts; and Fig. 3B, C, D, E and F show the activities of IE62s on the individual promoters of the gene 62 coding for IE62, the gene 28 for DNA polymerase, the gene 29 for major DNA binding protein (abbreviated as MDBP), the gene 14 for glycoprotein C (abbreviated as gC) and the gene 68 for gE, respectively.

### Best Mode for Carrying Out the Invention

Varicella virus gene 62 is one of the gene responsible for the transactivation function (function for activating transcription in the trans-form) and locates in the region of the base numbers 105142 to 109242 in the whole sequence of the varicella virus genome (Journal of General Virology, 67: 1759-1816, 1986). As shown in the upper column of Fig. 1, a transcription starts from the transcription start site along the arrow direction, so the nucleotide strand of the base numbers 109242 to 105142 in the whole genome sequence serves as "+" strand in the gene 62. Thus, the first base "A" in the SEQ ID No. 1 corresponds to the 5' terminal base (base at position 109242 in the whole genome sequence) of the + strand. In the following description, furthermore, base number is sometimes described as the base number in the whole genome sequence. Because the sequence of the gene 62 in the whole genome sequence is "-" strand, however, the gene 62 is complementary to the nucleotides shown in SEQ ID No. 1.

The gene 62 of Oka vaccine virus of the invention is a gene with the base substitutions (a) to (d) shown in Table 1 in the nucleotide sequence of SEQ ID No. 1. Otherwise, the gene 62 of Oka vaccine virus is a gene with one or more of the base substitutions (e) to (g) in Table 1 in addition to the base substitutions (a) to (d), or the gene additionally with one or more of the base substitutions (h) to (o) in Table 1.

The protein of the attenuated varicella virus of the invention is encoded by the gene 62, wherein the protein is with the amino acid substitutions (A) to (D) shown in Table 1 in the amino acid sequence of SEQ ID No. 1. Otherwise, the protein is with the amino acid substitutions of one or more of (E) to (H) shown in Table 1, in addition to the amino acid substitutions (A) to (D).

**Table 1**

| Base substitutions | Amino acid substitutions |
|---|---|
| (a) 2110th A → G | (A) 661st Ser → Gly |
| (b) 3100th A → G | (B) 958th Arg → Gly |
| (c) 3818th T → C | (C) 1197th Val → Ala |
| (d) 4006th A → G | (D) 1260th Ile → Val |
| (e) 1251st A → G | |
| (f) 2226th A → G | |
| (g) 3657th A → G | |
| (h) 162th T → C | |
| (i) 225th T → C | |
| (j) 523rd T → C | (E) 99th Met → Thr |
| (k) 1565th T → C | (F) 446th Leu → Pro |
| (l) 1763rd T → C | (G) 512nd Val → Ala |
| (m) 2652nd T → C | |
| (n) 4052nd T → C | (H) 1275th Leu → Ser |
| (o) 4193rd T → C | |

It is additionally suggested that the Oka vaccine virus carrying the gene 62 with the base substitutions causing such amino acid substitutions is attenuated probably due to the overall reduction of the expression of a protein required for the formation of virus particle because of the defection or attenuation of the transactivator function.

Such gene 62 of Oka vaccine virus can be isolated for example from the known Oka vaccine virus (BIKEN Lot-65: The Research Foundation of Microbial Diseases of Osaka University or ATCC VR-795) in a conventional manner. Otherwise, such Oka vaccine virus gene 62 can be isolated from an attenuated varicella-zoster virus strain identified by a method as described below.

The fragment of DNA of the present invention encodes the gene 62 set forth above, which includes the base-substituted portions, and which can be used as a probe for differentiating the attenuated varicella-zoster virus strains from wild-type varicella-zoster virus strains. The DNA fragment can be prepared by cleaving the genomic DNA of Oka vaccine virus or an attenuated varicella-zoster virus strain newly identified by the inventive method with appropriate restriction enzymes. The DNA fragment can be used for example as DNA probe for differentiating the Oka vaccine virus or the attenuated varicella-zoster virus strain from the highly toxic parental strain or other wild-type varicella-zoster virus strains.

Additionally, the protein of the present invention can be prepared by expressing the gene 62 in microorganisms for example *Escherichia coli* or yeast. More specifically, a DNA fragment carrying the coding region of the gene 62 is inserted in an expression vector with an origin replicable in microorganisms, a promoter, a ribosome-binding site, a DNA -cloning sites and a terminator to prepare an expression vector, to thereby transform a host cell; by subsequently culturing the resulting transformant, the protein encoded by the gene 62 can be produced in microorganisms at a mass scale. Otherwise, the gene 62 can be expressed in the form of a fusion protein with other proteins. By cleaving the resulting fusion protein with an appropriate protease, only the objective protein can be recovered.

These proteins can be used as antigens for preparing for example antibody and CTL.

The peptide of the present invention includes the amino acid substitutions in the amino acid sequence of the protein of the above (5) or (6). These peptides can be used as antigens for preparing antibodies and CTLs which recognise the protein of above (5) or (6) but not the protein encoded by gene 62 of wild-type varicella-zoster virus strains.

The antibody of the present invention can be prepared in the form of polyclonal antibody or monoclonal antibody using the protein per se or a partial peptide thereof as an antigen by a known method. Additionally, the CTL of the present invention can be prepared by using the protein per se or a partial peptide thereof as an antigen by a known method. These antibodies and CTL can be used for the method for identifying a newly isolated attenuated varicella-zoster virus strain.

The attenuated varicella-zoster virus strain mentioned herein has the identical base substitutions compared with those of the gene 62 of Oka vaccine virus. Therefore, the attenuated varicella-zoster virus strain mentioned herein is acceptable as a virus strain for live attenuated varicella vaccine. Such new attenuated varicella-zoster virus strains can be identified by sequencing the gene 62 of varicella-zoster viruses, and selecting a varicella virus carrying the same base substitutions in gene 62. More specifically, such new attenuated varicella-zoster virus strains can be differentiated from the wild-type varicella-zoster virus strain by the method using for example the DNA fragment or the antibody or CTL.

Otherwise, the new attenuated varicella-zoster virus strain can be identified by the method proposed in this application, namely the method comprising cleaving a DNA sequence comprising at least from 2107th to 2229th in the nucleotide sequence of SEQ ID No. 1 by using restriction enzymes *Nae*I and *Bss*HII, and selecting a varicella virus from which the DNA sequence is cleaved into 2 to 3 fragments. As has been described above, the base A at position 2110 in the gene 62 of Oka vaccine virus is substituted with G. Additionally, the base A at position 2226 is sometimes substituted with G. Consequently, the base substitution at position, an A vs. G, results in the creation of a restriction enzyme *Nae*I site (GCCGGC) at positions 2107 to 2112 in SEQ ID No. 1. Like that, the base substitution at position 2226, an A vs. G, makes *Bss*HII site (GCGCGC) at positions 2224 to 2229 in SEQ ID No. 1. In case that the DNA fragment at least comprising the nucleotides from 2107th to 2229th in SEQ ID No. 1 is cleaved with the two restriction enzymes, hence, the DNA fragment is cleaved into 2 or 3 fragments when the DNA fragment is derived from the Oka vaccine virus or the attenuated varicella virus. In case that the DNA sequence is derived from a wild-type varicella virus or the virulent parental strain of the Oka vaccine virus, while, the DNA sequence is never cleaved by either restriction enzymes, and remains as a single fragment because of no presence of such base substitutions as described above. The DNA fragment cleaved by the restriction enzymes can be prepared by PCR using the oligonucleotides, the nucleotide sequence of SEQ ID No. 2 and No. 3, as primers and the varicella virus gene 62 as an identification subject as template.

A live attenuated varicella vaccine may be prepared by using the attenuated varicella-zoster virus strain identified by the method described above as the seed virus. Such a live vaccine can be prepared in the same manner as the method for preparing conventional live attenuated varicella vaccines using the Oka vaccine virus as the seed virus.

### Examples

The invention will now be described specifically in detail in the following Examples but the invention is never limited to the following examples.

### Example 1

The nucleotide sequence of the gene 62 of Oka vaccine virus and the gene 62 of the highly toxic parental strain were determined. The Oka vaccine virus (live vaccine strain), Biken Lot-65: The research Foundation for Microbial Diseases of Osaka University was used. The virulent parental strain (wild-type Oka vaccine virus) was propagated in MRC-5 cells, and was then used.

According to the method described in the Example 1 of the previous invention (WO 97/43420), more specifically, genomic DNA were extracted from the Oka vaccine virus and the virulent parental strain, and each DNA was amplified by PCR using primers designed by refering to the nucleotide sequence of the Dumas strain (J. Gen. Virol., 67: 1759-1816, 1986). The entire gene 62 was amplified as three entire overlapping pieces using three sets of primer pair (sense primers G62N1, G62N2 and G62N3 and antisense primers G62R1, G62R2 and G62R3) as shown in Table 2. Each sense primer had M13 forward (-38) sequence (nucleotides at positions 1 to 19 in G62N1-3) at their 5' end, and each antisense primer had M13 reverse sequence (nucleotides at positions 1 to 20 in G62R1-3) linked at their 5' end.

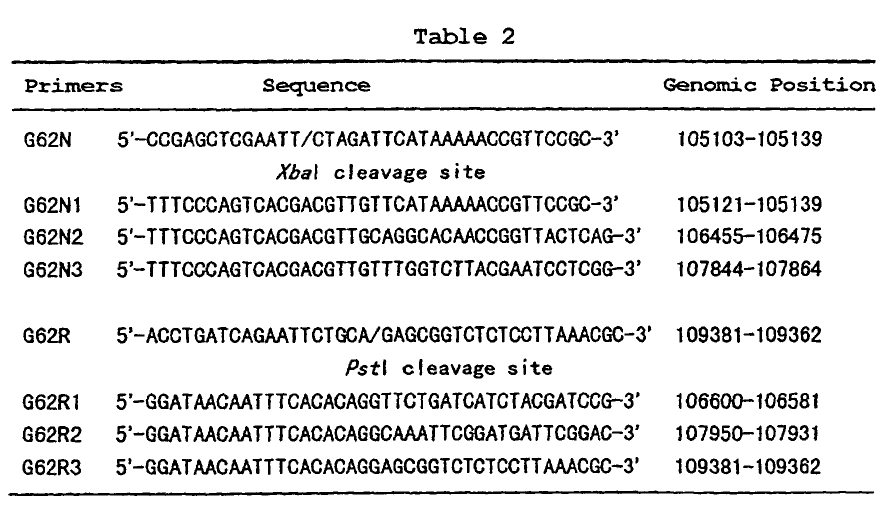

Each PCR reaction consisted of 200 mM of deoxynucleotide triphosphate [each], 0.3 µM of each primer, an extremely low amount of template DNA, and 2.5 U of Ex Taq (manufactured by TaKaRa) in 50 µl of Ex Taq buffer. One set of primer pairs (SEQ ID Nos. 3 and 8) included 6 % DMSO in the reaction mixture. Amplification was carried out at 30 cycles of denaturation (94 °C for one minute), annealing (55 °C for 1.5 minutes) and extension (72 °C for 2 minutes), using thermal cycler (Perkin-Elmer, USA).

All PCR products were purified using the PCR purification kit (QIAGEN GmbH, Germany) to remove primers. Direct sequence of the purified PCR products was performed with a sequencing kit (Amersham, Co., UK) using M13 forward primer or reverse primer labeled with a fluorescent dye IRO-40, analyzed using a DNA sequencer Model 4000L (LI-COR Co., USA).

By the same method, the DNA sequence of genes 4, 14 and 61 of Oka vaccine virus and the virulent parental strain were determined.

Consequently, there were no nucleotide change in the genes 4, 14 and 61 between the Oka vaccine virus and the virulent parental strain. As shown in Table 3, 15 bases differences were found in the gene 62 between Oka vaccine virus and the virulent parental strain. Every base substitutions was a T vs C or an A vs G. At eight positions, the nucleotide changes found in the vaccine virus resulted from mixture of two kinds of nucleotide at a single position (R in Table 3). From the respect of 9 bases, additionally, the gene 62 of each of the Oka vaccine virus and the virulent parental strain was different from the gene 62 of the Dumas strain.

Based on the aforementioned results, it was confirmed that the attenuated Oka vaccine virus could be differentiated from the virulent parental strain in terms of the sequence difference of gene 62.

**Table 3**

| Genomic Position | Vaccine Strain | Parental Strain | Dumas Strain |
|---|---|---|---|
| 105169 | R (noncoding) | A (noncoding) | A |
| 105310 | R (Ser/Leu) | A (Leu) | A |
| 105356 | C (Val) | T (Ile) | T |
| 105451 | G | G | A |
| 105512 | C | C | A |
| 105544 | G (Ala) | A (Ala) | A |
| 105705 | C (Ala) | T (Ala) | T |
| 106262 | C (Gly) | T (Arg) | T |
| 106710 | R (Ala) | A (Ala) | A |
| 107136 | C (Ala) | T (Ala) | T |
| 107165 | T | T | C |
| 107252 | C (Gly) | T (Ser) | T |
| 107303 | C | C | T |
| 107599 | R (Ala/Val) | A (Val) | A |
| 107607 | A | A | C |
| 107715 | C | C | T |
| 107797 | R (Pro/Leu) | A (Leu) | A |
| 108111 | C (Pro) | T (Pro) | T |
| 108747 | G | G | A |
| 108838 | R (Thr/Met) | A (Met) | A |
| 108951 | A | A | G |
| 109044 | G | G | C |
| 109137 | R (silent) | A (silent) | A |
| 109200 | R (silent) | A (silent) | A |

### Example 2

The entire gene 62 was cloned from Oka vaccine virus and the virulent parental strain by PCR.

PCR conditions were same as in Example 1 except for the use of the oligonucleotides G62N and G62R in Table 1 as primer pairs, the addition of 6 % DMSO in the reaction mixture, and an extension time of 6 minutes for every cycle. The full length of each gene 62 was amplified by PCR.

Because the primers individually had restriction enzymes *Xba*I and *Pst*I sites, the purified PCR product was digested with these restriction enzymes. And then the digested product was inserted into pUC19, which had been digested in advance with the corresponding enzymes. After the transformation of *E. coli* JM109 with hybrid plasmids, 9 plasmids were extracted from the Oka vaccine and the virulent parental transformants for DNA sequencing.

The results are shown in Fig. 1. As shown in Fig. 1, plural bases among the bases at 15 positions in each of the 9 clones of the Oka vaccine virus were different from those of the virulent parental strain. Particularly, the 7 bases at base positions 1251, 2110, 2226, 3100, 3657, 3818 and 4006 in all of the clones were different from the bases at the same positions in the parental strain. No nucleotide changes were observed in any of the 9 clones from the virulent parental strains for these 15 sites.

### Example 3

Partial sequences of the gene 62s of various varicella-zoster virus strains were analyzed by RFLP.

The Oka vaccine virus and the virulent parental strain were used as way in Example 1. Additionally, the Kawaguchi strain, one of wild-type varicella-zoster virus strains and 10 samples of wild-type virus strains isolated from vesicular fluids from non-vaccinated 5 patients with varicella and 5 patients with zoster were used. Herein, the Kawaguchi strain and the wild-type varicella-zoster virus strains isolated from these patients were propagated in HEL cell or MRC-5 cell over 2 to 3 times, and were then used.

The gene 62 of each varicella virus was amplified by PCR using the oligonucleotides of SEQ ID Nos. 2 and 3 as primers. The oligonucleotide of SEQ ID No. 2 was the reverse primer and corresponding to the bases at positions 1846 to 1863 in the varicella virus gene 62 (SEQ ID No. 1), while the oligonucleotide of SEQ ID No. 3 was the forward primer corresponding to the bases at positions 2609 to 2620 thereof. PCR conditions were the same as in Example 1, except that the extension time was one minute for every cycle. Three microliters of each PCR product were digested sequentially with 4 U *Nae*I (TaKaRa) at 37 °C for 1.5 hours and 4U *Bss*HII (TaKaRa) at 50 °C for 1.5 hours. The digested DNA fragments were analyzed by electrophoresis on 4 % agarose gel (NuSieve 3:1, FMC BioProducts, Co.) with ethidium bromide staining.

The results are shown in Fig. 2. As apparently shown in Fig. 2, the PCR product (Lane 1) of the gene 62 from Oka vaccine virus was cleaved into three fragments (402 bp, 264 bp and 116 bp) by *Nae*I and *Bss*HII, whereas the PCR products (Lanes 2-13) of the virulent parental strain and the wild-type virus strains isolated from the patients were never digested by either restriction enzymes and remained as a single fragment.

The aforementioned results establish the confirmation that by cleaving a DNA sequence at least comprising the base at position 2107 to 2229 in the varicella virus gene 62 with restriction nucleases *Nae*I and *Bss*HII, a varicella virus with a DNA sequence thereby cleaved into 2 to 3 fragments could be identified as the Oka vaccine virus or a virus strain acceptable as attenuated varicella virus.

### Example 4

So as to analyze the correlation between the eight amino acid mutations ad the transactivation activities f the gene 62 products, IE62, chloramphenicol transferase (CAT) assays were performed in cells cotransfected with a reporter plasmid having a promoter sequence of each VZV gene upstream of CAT gene and an effector plasmid carrying the gene 62 of Oka vaccine virus or the highly toxic parental strain.

### 1. Materials and Methods

### 1.1 Plasmids

Reporter plasmids pIE4-CAT, pgC-CAT, pPOL-CAT, pMDBP-CAT, pIE62-CAT and pgE-CAT contained about 750 bases upstream of the initiation codon the promoter region of genes 4, 14, 28, 29, 62 and 68, respectively. Each promoter region was amplified from the Oka vaccine strain by PCR using specific primers which had *Nhe*I- or *Bgl*II site at the 5' end. Conditions of PCR were the same as in Example 1, except for the extension time of one minute. The amplified products were digested with *Nhe*I and *Bgl*II, and were then inserted into upstream of CAT gene of pCAT3-Basic plasmid (Promega, Co., USA).

Effector plasmid pPar-G62 was selected among 9 clones from gene 62 clone of the virulent parental strain. Clone 9 of Fig. 1 (the clone containing 13 base substitutions and 8 amino acid substitutions) was selected from among the 9 clones from the Oka vaccine strain and uses as the effector plasmid, pVac-G62.

### 1.2 Transfection

CV1 cells were cultured in a 35-mm plastic dish at 10⁵ cells/dish and were transfected by lipofection using Superfect (QIAGEN GmbH). Each reaction mixture for transfection consisted of 0.25 µg of reporter plasmid and various amounts (0-1 µg) of either effector plasmid . The total DNA amount in each transfection was kept constant at 2.5 µg by adding the vector pUC19. All experiments were repeated at least three times, with independent DNA transfection.

### 1.3 CAT assay

Forty four hours after transfection, the total protein and CAT levels in the cell were assayed. The cell was washed three times with phosphate-buffered saline, and then lysed in lysis buffer (Boehringer Mannheim Corporation, Germany). The CAT concentration of the individual lysate was determined by a CAT ELISA kit (Boehringer Mannheim Corporation, Germany) and was standardized to each protein concentration, that was determined by the Bio-Rad protein reagent (Bio-Rad, USA).

### 2. Results

All of the VZV gene promoters tested displayed very low-level basal activity; little or no CAT expression was observed when no effector plasmid was transfected.

The gene 4 promoter derived from Oka strain was transactivated by both gene 62 products (IE62s) of the virulent parental strain and the Oka strain (Fig. 3A). The transactivation activity of IE62 of the virulent parental strain was stronger than that of the IE62 by the Oka vaccine strain. When the lowest dose (0.25 µg) of the effector plasmids was transfected, the activity of the virulent parental strain IE62 was 7.8-fold higher than that by the Oka strain IE62. As the effector concentration was increased, the difference in the activity of the two IE62 proteins diminished.

Little CAT could be detected in the cells transfected with the reporter plasmid pCAT-IE62 (Fig. 3B).

Furthermore, pCAT-Pol and pCAT-MDBP carrying the VZV genes 28 and 29 promoters, respectively (Fig. 3C and 3D), and pCAT-gE carrying the promoter sequence of the gene 68 (Fig. 3F) were activated by both IE62s from the virulent parental strain and the Oka vaccine strain. Each dose-dependent curves was similar to the pCAT-IE4 response, and the activity of the virulent parental strain IE62 was higher than that of the Oka vaccine strain IE62 at all dose. At the lowest dose of effector plasmids, in particular, the activity of the virulent parental strain IE62 was 7.6-fold, 5.6-fold and 1.8-fold higher than that of the Oka vaccine strain IE62. Little CAT activity was detected in the cell transfected with pCAT-gC carrying the promoter region of the gene 14 (Fig. 3E).

The aforementioned results establish the confirmation that the expression product (IE62) of the VZV gene 62 activated the promoters of the pre-early gene 4 (ORF4), early gene 28 (DNA polymerase: Pol) and gene 29 (major DNA binding protein: MDBP) and delayed gene 68 (glycoprotein: gE). It is additionally of more importance that the transcription activity of the Oka vaccine strain IE62 was constantly lower than that of the highly toxic parental strain IE62. In other words, the mutations in the gene 62 of the Oka vaccine strain have some influence on virus replication, which induces the attenuation of VZV.

### Industrial Applicability

In accordance with the present invention, the Oka vaccine virus or a varicella-zoster virus strain acceptable as attenuated varicella vaccine can be accurately discriminated in a simple manner from other wild-type varicella viruses and the highly toxic parental strain of the Oka vaccine virus. In accordance with the present invention, furthermore, attenuated varicella virus antigens for live vaccine can be prepared at a mass scale. Owing to the inventions, vaccine preparation and the safety and effectiveness of vaccination can be achieved in a more reliable manner.

## Claims

1. Gene 62 of Oka vaccine virus, which comprises the following base substitutions (a) to (d) in the nucleotide sequence of SEQ ID No. 1:
(a) the base A at position 2110 is G;
(b) the base A at position 3100 is G;
(c) the base T at position 3818 is C; and
(d) the base A at position 4006 is G.

2. The gene 62 of the Oka vaccine virus of claim 1, which comprises one or more of the following base substitutions (e) to (g) in addition to the base substitutions (a) to (d):
(e) the base A at position 1251 is G;
(f) the base A at position 2226 is G; and
(g) the base A at position 3657 is G.

3. The gene 62 of the Oka vaccine virus of claim 2, which comprises one or more of the following base substitutions (h) to (o), in addition to the base substitutions (a) to (d) and one or more of the base substitutions (e) to (g);
(h) the base T at position 162 is C;
(i) the base T at position 225 is C;
(j) the base T at position 523 is C;
(k) the base T at position 1565 is C;
(l) the base T at position 1763 is C;
(m) the base T at position 2652 is C;
(n) the base T at position 4052 is C; and
(o) the base T at position 4193 is C.

4. A fragment of DNA encoding the gene 62 of any one of claims 1 to 3, which includes the base-substituted portions, and which can be used as a probe for differentiating the attenuated varicella-zoster virus strains from wild-type varicella-zoster virus strains.

5. A protein encoded by the gene 62 of the Oka vaccine virus of claim 1, which comprises the following amino acid substitutions (A) to (D) in the amino acid sequence of SEQ ID No. 1:
(A) the amino acid residue Ser at position 628 is Gly;
(B) the amino acid residue Arg at position 958 is Gly;
(C) the amino acid residue Val at position 1197 is Ala; and
(D) the amino acid residue Ile at position 1260 is Val.

6. The protein of claim 5, which comprises one or more of the following amino acid substitutions (E) to (H) in addition to the amino acid substitutions (A) to (D):
(E) the amino acid residue Met at position 99 is Thr;
(F) the amino acid residue Leu at position 446 is Pro;
(G) the amino acid residue Val at position 512 is Ala; and
(H) the amino acid residue Leu at position 1275 is Ser.

7. A peptide as a part of the protein of claim 5 or 6, which includes the amino acid-substituted portions, and which can be used as an antigen for preparing an antibody or a cytotoxic T lymphocyte which recognises the protein of claim 5 or claim 6 but not the protein encoded by gene 62 of wild-type varicella-zoster virus strains.

8. An antibody or a cytotoxic T lymphocyte which recognises the protein of claim 5 or 6 or the peptide of claim 7 but does not recognise the protein of gene 62 of wild-type varicella-zoster virus strains.

9. A method for identifying the Oka vaccine virus or an attenuated varicella-zoster virus strain, comprising sequencing the gene 62 of varicella-zoster viruses, and selecting a varicella-zoster virus with the same base substitutions in the gene 62 of any one of claims 1 to 3.

10. A method for identifying the Oka vaccine virus or an attenuated varicella-zoster virus strain, comprising selecting a varicella-zoster virus carrying genomic DNA to which the DNA fragment of claim 4 hybridizes.

11. A method for identifying the Oka vaccine virus or an attenuated varicella-zoster virus strain, comprising selecting a varicella-zoster virus producing an antigen recognised by the antibody or the cytotoxic T lymphocyte of claim 8.

12. A method for identifying the Oka vaccine virus or an attenuated varicella-zoster virus strain, comprising digesting a DNA fragment comprising at least from 2107th to 2229th in the nucleotide sequence of SEQ ID No. 1 by using restriction enzymes NaeI and BssHII, and selecting a varicella-zoster virus from which the DNA fragment is digested into 2 or 3 fragments.

13. A method according to claim 12, wherein the DNA sequence is prepared by PCR using the oligonucleotides of the nucleotide sequence of SEQ ID No. 2 and 3 as primers and varicella-zoster virus gene 62 as a template.

## Patentansprüche

1. Gen 62 des Oka-Vakzin-Virus, das die folgenden Basensubstitutionen (a) bis (d) in der Nukleotidsequenz SEQ ID NO: 1 umfaßt:
(a) die Base A in Position 2110 ist G;
(b) die Base A in Position 3100 ist G;
(c) die Base T in Position 3818 ist C und
(d) die Base A in Position 4006 ist G.

2. Gen 62 des Oka-Vakzin-Virus nach Anspruch 1, das eine oder mehrere der folgenden Basensubstitutionen (e) bis (g) zusätzlich zu den Basensubstitutionen (a) bis (d) umfaßt:
(e) die Base A in Position 1251 ist G;
(f) die Base A in Position 2226 ist G und
(g) die Base A in Position 3657 ist G.

3. Das Gen 62 des Oka-Vakzin-Virus nach Anspruch 2, das eine oder mehrere der folgenden Basensubstitutionen (h) bis (o) zusätzlich zu den Basensubstitutionen (a) bis (d) und einer oder mehreren der Basensubstitutionen (e) bis (g) umfaßt:
(h) die Base T in Position 162 ist C;
(i) die Base T in Position 225 ist C;
(j) die Base T in Position 523 ist C;
(k) die Base T in Position 1565 ist C;
(l) die Base T in Position 1763 ist C;
(m) die Base T in Position 2652 ist C;
(n) die Base T in Position 4052 ist C und
(o) die Base T in Position 4193 ist C.

4. DNA-Fragment, das für das Gen 62 nach einem der Ansprüche 1 bis 3 codiert, das die Basen-substituierten Teile enthält und das als Sonde zur Differenzierung der abgeschwächten Varicella-Zoster-Virus-Stämme von Wildtyp-Varicella-Zoster-Virus-Stämmen eingesetzt werden kann.

5. Protein, das durch das Gen des Oka-Vakzin-Virus nach Anspruch 1 codiert wird, das die folgenden Aminosäuresubstitutionen (A) bis (D) in der Aminosäuresequenz SEQ ID NO: 1 umfasst:
(A) der Aminosäurerest Ser in Position 628 ist Gly;
(B) der Aminosäurerest Arg in Position 958 ist Gly;
(C) der Aminosäurerest Val in Position 1197 ist Ala und
(D) der Aminosäurerest Ile in Position 1260 ist Val.

6. Protein nach Anspruch 5, das eine oder mehrere der folgenden Aminosäuresubstitutionen (E) bis (H) zusätzlich zu den Aminosäuresubstitutionen (A) bis (D) umfaßt:
(E) der Aminosäurerest Met in Position 99 ist Thr;
(F) der Aminosäurerest Leu in Position 446 ist Pro;
(G) der Aminosäurerest Val in Position 512 ist Ala und
(H) der Aminosäurerest Leu in Position 1275 ist Ser.

7. Peptid als Teil des Proteins nach Anspruch 5 oder 6, das die Aminosäure-substituierten Teile enthält und das als Antigen zur Herstellung eines Antikörpers oder eines cytotoxischen T-Lymphozyten verwendet werden kann, der das Protein nach Anspruch 5 oder 6 erkennt, aber nicht das Protein, das durch Gen 62 der Wildtyp-Varicella-Zoster-Virus-Stämme codiert wird.

8. Antikörper oder cytotoxischer T-Lymphozyt, der das Protein nach Anspruch 5 oder 6 oder das Peptid nach Anspruch 7 erkennt, nicht aber das Protein von Gen 62 der Wildtyp-Varicella-Zoster-Virus-Stämme erkennt.

9. Verfahren zur Identifizierung des Oka-Vakzin-Virus oder eines abgeschwächten Varicella-Zoster-Virus-Stamms, umfassend Sequenzieren des Gens 62 von Varicella-Zoster-Viren und Selektieren eines Varicella-Zoster-Virus mit denselben Basensubstitutionen in Gen 62 nach einem der Ansprüche 1 bis 3.

10. Verfahren zur Identifizierung des Oka-Vakzin-Virus oder eines abgeschwächten Varicella-Zoster-Virus-Stamms, umfassend Selektieren eines Varicella-Zoster-Virus, das genomische DNA trägt, an die das DNA-Fragment nach Anspruch 4 hybridisiert.

11. Verfahren zur Identifizierung des Oka-Vakzin-Virus oder eines abgeschwächten Varicella-Zoster-Virus-Stamms, umfassend Selektieren eines Varicella-Zoster-Virus, das ein Antigen produziert, das durch den Antikörper oder den cytotoxischen T-Lymphozyten von Anspruch 8 erkannt wird.

12. Verfahren zur Identifizierung des Oka-Vakzin-Virus oder eines abgeschwächten Varicella-Zoster-Virus-Stamms, umfassend Verdauen eines DNA-Fragments, das wenigstens vom 2107. bis 2229. in der Nukleotidsequenz SEQ ID NO: 1 umfaßt, durch Verwendung der Restriktionsenzyme NaeI und BssHII und Selektieren eines Varicella-Zoster-Virus, bei dem das DNA-Fragment in zwei oder drei Fragmente verdaut ist.

13. Verfahren nach Anspruch 12, wobei die DNA-Sequenz durch PCR unter Verwendung der Oligonukleotide der Nukleotidsequenz SEQ ID NO: 2 und 3 als Primer und Varicella-Zoster-Virus-Gen 62 als Matrize hergestellt wird.

## Revendications

1. Gène 62 du virus vaccinal Oka, qui comprend les remplacements de bases (a) à (d) suivants dans la séquence nucléotidique de SEQ ID No. 1 :
(a) la base A à la position 2110 est G ;
(b) la base A à la position 3100 est G ;
(c) la base T à la position 3818 est C ; et
(d) la base A à la position 4006 est G.

2. Gène 62 du virus vaccinal Oka selon la revendication 1 qui comprend un ou plusieurs des remplacements de bases (e) à (g) suivants en plus des remplacements de bases (a) à (d) :
(e) la base A à la position 1251 est G ;
(f) la base A à la position 2226 est G ; et
(g) la base A à la position 3657 est G.

3. Gène 62 du virus vaccinal Oka selon la revendication 2 qui comprend un ou plusieurs des remplacements de bases (h) à (o) suivants, en plus des remplacements de bases (a) à (d) et d'un ou plusieurs des remplacements de bases (e) à (g) ;
(h) la base T à la position 162 est C ;
(i) la base T à la position 225 est C ;
(j) la base T à la position 523 est C ;
(k) la base T à la position 1565 est C ;
(l) la base T à la position 1763 est C ;
(m) la base T à la position 2652 est C ;
(n) la base T à la position 4052 est C ; et
(o) la base T à la position 4193 est C.

4. Fragment d'ADN codant le gène 62 selon l'une quelconque des revendications 1 à 3, qui inclut les portions à bases remplacées et qui peut être utilisé comme sonde pour différentier les souches d'herpèsvirus varicella atténuées des souches d'herpèsvirus varicella de type sauvage.

5. Protéine codée par le gène 62 du virus vaccinal Oka selon la revendication 1, qui comprend les remplacements d'aminoacides (A) à (D) suivants dans la séquence d'aminoacides de SEQ ID No. 1 :
(A) le résidu d'aminoacide Ser à la position 628 est Gly ;
(B) le résidu d'aminoacide Arg à la position 958 est Gly ;
(C) le résidu d'aminoacide val à la position 1197 est Ala ; et
(D) le résidu d'aminoacide Ile à la position 1260 est Val.

6. Protéine selon la revendication 5, qui comprend un ou plusieurs des remplacements d'aminoacides (E) à (H) suivants en plus des remplacements d'aminoacides (A) à (D) :
(E) le résidu d'aminoacide Met à la position 99 est Thr ;
(F) le résidu d'aminoacide Leu à la position 446 est Pro ;
(G) le résidu d'aminoacide Val à la position 512 est Ala ; et
(H) le résidu d'aminoacide Leu à la positon 1275 est Ser.

7. Peptide à titre de partie de la protéine selon la revendication 5 ou 6, qui indut les portions à remplacements d'aminoacides, et qui peut être utilisé comme antigène pour préparer un anticorps ou un lymphocyte T cytotoxique qui reconnaît la protéine selon la revendication 5 ou la revendication 6 mais pas la protéine codée par le gène 62 de souches d'herpèsvirus varicella de type sauvage.

8. Anticorps ou lymphocyte T cytotoxique qui reconnaît la protéine selon la revendication 5 ou 6 ou le peptide selon la revendication 7 mais qui ne reconnaît pas la protéine du gène 62 de souches d'herpèsvirus varicella de type sauvage.

9. Procédé pour identifier le virus vaccinal Oka ou une souche d'herpèsvirus varicella atténuée, comprenant le séquençage du gène 62 d'herpèsvirus varicella, et la sélection d'un herpèsvirus varicella ayant les mêmes remplacements de bases dans le gène 62 selon l'une quelconque des revendications 1 à 3.

10. Procédé pour identifier le virus vaccinal Oka ou une souche d'herpèsvirus varicella atténuée, comprenant la sélection d'un herpèsvirus varicella portant un ADN génomique auquel le fragment d'ADN selon la revendication 4 s'hybride.

11. Procédé pour identifier le virus vaccinal Oka ou une souche d'herpèsvirus varicella atténuée, comprenant la sélection d'un herpèsvirus varicella produisant un antigène reconnu par l'anticorps ou le lymphocyte T cytotoxique selon la revendication 8.

12. Procédé pour identifier le virus vaccinal Oka ou une souche d'herpèsvirus varicella atténuée, comprenant la digestion d'un fragment d'ADN comprenant au moins du 2107^{ème} au 2229^{ème} dans la séquence nucléotidique de SEQ ID No. 1 au moyen des enzymes de restriction NaeI et BssHII, et la sélection d'un herpèsvirus varicella dont le fragment d'ADN est digéré en 2 ou 3 fragments.

13. Procédé selon la revendication 12, où la séquence d'ADN est préparée par PCR avec les oligonucléotides de la séquence nucléotidique de SEQ ID No. 2 et 3 comme amorces et le gène 62 d'herpèsvirus varicella comme matrice.
